(19) Europäisches Patentamt / European Patent Office / Office européen des brevets

(11) **EP 4 025 676 B1**

(12) **EUROPEAN PATENT SPECIFICATION**

(45) Date of publication and mention
of the grant of the patent:
**02.08.2023 Bulletin 2023/31**

(21) Application number: **20764105.1**

(22) Date of filing: **02.09.2020**

(51) International Patent Classification (IPC):
***C11B 13/00*** (2006.01)  ***C07J 9/00*** (2006.01)

(52) Cooperative Patent Classification (CPC):
**C11B 13/005; C07J 9/00;** Y02W 30/74

(86) International application number:
**PCT/EP2020/074500**

(87) International publication number:
**WO 2021/043845 (11.03.2021 Gazette 2021/10)**

(54) **PRODUCTION OF AN EXTRACT OF PHYTOSTEROLS AND STANOLS FROM TALL OIL PITCH**

HERSTELLUNG EINES EXTRAKTS VON PHYTOSTERINEN UND STANOLEN AUS TALLÖLPECH

PRODUCTION D'UN EXTRAIT DE PHYTOSTÉROLS ET DE STANOLS À PARTIR DE BRAI DE TALLÖL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.09.2019 LU 101374**

(43) Date of publication of application:
**13.07.2022 Bulletin 2022/28**

(73) Proprietor: **Pine Chemical Holding S.A.**
**1251 Luxembourg (LU)**

(72) Inventors:
• **TSEBULAEV, Victor**
**4970 Bettange/Mess (LU)**
• **KHODOV, Nikolay**
**8086 Bertrange (LU)**
• **RADBIL, Arkady**
**Nizhniy Novgorod, 603098 (RU)**
• **DOLINSKI, Taras**
**Nizhniy Novgorod, 603146 (RU)**
• **KORSHUNOV, Alexey**
**Nizhniy Novgorod Region,**
**Sarov, 607188 (RU)**
• **CHERNOV, Ilya**
**Nizhniy Novgorod region, District Ourenskyi, Ousta, 606822 (RU)**
• **LAZAREV, Mikhail**
**Nizhniy Novgorod, 603159 (RU)**

(74) Representative: **Mellet, Valérie Martine et al**
**Patent 42**
**5, rue Dicks**
**4081 Esch-sur-Alzette (LU)**

(56) References cited:
US-A- 4 265 824  US-A- 4 279 827
US-A1- 2004 024 175  US-A1- 2004 267 032
US-A1- 2011 034 725  US-A1- 2013 261 284

• WANG SHI-FA ET AL: "Study of extraction of phytosterol from masson pine raw tall oil", JOURNAL OF WOOD SCIENCE, SPRINGER JAPAN KK, JP, vol. 48, no. 6, 1 December 2002 (2002-12-01), pages 505-511, XP036418171, ISSN: 1435-0211, DOI: 10.1007/BF00766647 [retrieved on 2002-12-01]

**Description**

**Field of the disclosure**

The present disclosure relates to a process for the production of an extract of phytosterols and stanols from tall oil pitch.

**Background of the disclosure**

Phytosterols and stanols can be found in almost all plants and are usually commercialized under the form of an extract. In this kind of extract, β-sitosterol is one of the main subcomponents and is very similar in composition to cholesterol. β-sitosterol, or derivatives thereof, when administered to subjects, provides a hypocholesterolemic effect.
The idea of recovering β-sitosterol from plants is therefore interesting to develop raw material in the production of pharmaceutical preparations and/or nutritional products helping the decrease in cholesterol blood level. To obtain β-sitosterol from plants, it is necessary to isolate the phytosterol content that can be found in the plants by way of different techniques, including distillation, extraction and/or crystallization. Such techniques often lead to sterol concentrates, that comprise β-sitosterol, but also α-sitosterol, campesterol, stigmasterol, brassicasterol, β-sitostanol, campestanol, oxysterols, triterpene alcohols, as well as other components, such as fatty alcohols. During the process of isolating β-sitosterol, other components must also be removed, such as fibres, lignin materials and inorganic salts.
A good source of phytosterols is crude tall oil, which is a by-product of the Kraft process of wood pulp manufacture. Rectification of crude tall oil affords tall oil pitch as a residue that can be then treated to afford a sterol concentrate. Tall oil pitch is composed of esters of fatty and rosin acids, their oligomers and oxidized derivatives, high-boiling neutral substances (hydrocarbons, alcohols, including phytosterols, *etc*...) and lignin.
US 6,465,665 describes a continuous process for the recovery of sterols from a mixture of neutral matter obtained from tall oil pitch. The mixture of the neutral matter is obtained through saponification and extraction with petroleum ether of tall oil pitch. This method allows recovering neutral matter with a content of sterols amounting to 41.8%. Following the recovery of the neutral matter, its distillation through a rectifying column is undertaken to obtain a first fraction of long-chain aliphatic alcohol and a second fraction of sterols and esters. A second distillation is performed on the second fraction to separate the sterols and the esters. The sterols fraction is then dissolved into essentially liquid hydrocarbons but with also potentially alcohol and/or water. The solution is then cooled down to form a precipitate which is separated from the mother liquor. The mother liquor is evaporated to form a residue which is added to the mixture of the neutral matter before its distillation. In conclusion, from tall oil pitch, the yield of sterol that was recovered according to the described process was 94.7% and the purity of the sterols is higher than 95%. This method, however, does not describe the way to obtain β-sitosterol or even a mixture comprising a high content of β-sitosterol.
US 3,965,085 describes a method for refining soaps in which the unsaponifiable neutral substances, which are known to drastically impair the quality of the crude tall oil, are separated by means of extraction using low molecular weight ketones (such as acetone and/or methyl ethyl ketone) in combination with water-immiscible solvents like liquid hydrocarbons. The low molecular weight ketones can indeed prevent the formation of emulsions in soap solutions and are easy to remove, even in acidic conditions. During the extraction, the organic phase contains the unsaponifiables and the water phase contains the salts of fatty acids and rosin acids. The organic phase and the water phase can be then separated, which allows recovering refined tall oil with a content of neutral substances as low of 1.6%. Further treatment of the organic phase, such as evaporation and crystallization, allows the recovery of a sterol concentrate with a content of β-sitosterol of at least 80%. Nevertheless, the study mentions that the sterol concentrate also comprises campesterol, fatty alcohols and triterpene alcohols.
WO 2019/050430 describes a method for extracting phytosterols from tall oil pitch. The method comprises a step of saponification of the tall oil pitch using an alkali in polyatomic alcohol. Unsaponifiables are then collected and extracted from the alkali-alcohol solution using a mixture of paraffin hydrocarbons as the solvent. After removal of the solvent by distillation, betulin, which is a triterpene alcohol and a cell poison, has been isolated by crystallization and the phytosterols have been concentrated by means of rectification. This method has thus provided an end product which has a phytosterol content of at least 65% with unwanted betulin impurities at not more than 0.3%. Although this method allows for obtaining a sterol concentrate free of one particular component (i.e. betulin), the extraction of β-sitosterol from tall oil pitch in elevated content is still not reached since this document relates only to a high degree of extraction of phytosterols in general. Additional components should be removed to obtain an elevated content in β-sitosterol in an end product that could then be valorised as pharmaceutical preparation and/or nutritional product.
US 2008/0161586 describes a process for recovering sterols from tall oil pitch. After producing a mixture of neutral substances, the fatty alcohols are removed by distillation. The non-volatile residue is then crystallized at a temperature comprised between 50°C and 70°C using hydrocarbon solvent, alcohols and water. In the example of this disclosure, gas chromatography (GC) analysis of the dried sterols showed an extract having 86.7% of purity (in sterols and stanols) with near 10% of fatty alcohols (wax). Among the 86.7% of sterols and stanols, there are 6.3% of campesterol, 1% of ergostanol, 1% of stigmasterol, 69.5% of $\beta$-sitosterol and 9% of $\beta$-sitostanol (stigmastanol).

US 2011/034725 describes a process for recovering sterols and fatty and/or resin acids from tall oil pitch, in which a crystallization step using a mixture of solvents comprising at least one or two of the solvents selected from the group consisting of ketones, alkanols, hydrocarbons and water is performed.

US 2004/267032 describes a process for isolating phytosterols from a pulping soap comprising among other things an extraction step achieved by using polar solvents.

US 2004/024175 describes a method of preparing phytosterols from tall oil pitch containing steryl esters.

US 4 279 827 describes a process for the preparation of a $\beta$-sitosterol concentrate containing less than 5% by weight of $\alpha$-sitosterol from sitosterol concentrates derived from plants by treating the sitosterol concentrate with a solvent mixture containing aromatic and/or aliphatic hydrocarbons as well as esters and, in addition, possible small amounts of other solvents.

US 2013/261284 describes a process for recovering fatty acids, resin acids and sterols from tall oil pitch.

The objective of the present disclosure is therefore to provide a process for improving the production extract of phytosterols and stanols from tall oil pitch.

**Summary of the disclosure**

According to a first aspect, the disclosure provides a process for providing an extract of phytosterols and stanols from tall oil pitch, the process comprising the following steps:

> a) providing a tall oil pitch;
> b) saponifying the tall oil pitch to a produce saponified tall oil pitch by addition of a basic compound to the tall oil pitch;
> c) extracting the saponified tall oil pitch with one or more apolar aromatic solvents, to produce a solution comprising a mixture of neutral matters;
> d) performing a distillation of the mixture of neutral matters to obtain a distillate fraction and a bottom fraction;
> e) dissolving the distillate fraction into mixed solvents, to obtain a dissolved distillate fraction,

the process is remarkable in that it further comprises a step (f) of crystallization which is performed on the dissolved distillate fraction under crystallization conditions to recover a mother solution and a crystallized fraction, wherein the crystallized fraction forms an extract of phytosterols and stanols; and in that the mixed solvents used on step (e) comprise one or more apolar solvents, one or more solvents having a ketone moiety, one or more monohydric alcohols and water.

Surprisingly, it has been found that when starting from a mixture of neutral matters obtained from a tall oil pitch, it was possible to obtain an extract of phytosterols and stanols that has an improved high content in $\beta$-sitosterol with an improved low amount of impurities. The mixture of neutral matters is first purified by distillation to obtain a volatile distillate fraction. The volatile distillate fraction is dissolved in a mixture of solvents and submitted to a crystallization step. The specific mixture of solvent used according to the disclosure renders the crystallization very efficient. As a consequence, it is possible to obtain a final extract of phytosterols and stanols containing an improved content of major sterols and stanols. Unwanted impurities such as $\alpha$-sitosterol, and oxysterols are removed during the crystallization step. Moreover, no triterpene alcohol (*e.g.* betulin) has been detected in the final extract. Last but not least, the process of the disclosure allows for having a high content of $\beta$-sitosterol into the extract.

About the crystallization step, it has been found that a solvent being a mixture of at least four solvents was to be used to obtain the requested purified extract. Indeed, the apolar solvent allows for dissolving the di- and triterpenic hydrocarbons and condensed products that come from the tall oil pitch. The ketone moiety is a substance capable to dissolve $\alpha$-sitosterol. The monohydric alcohols are dissolving the sterols. Water is triggering the crystallization of the sterols.

In a preferred embodiment, step (c) of extracting the saponified tall oil pitch with one or more apolar aromatic solvents is carried out at a temperature ranging between 80°C and a temperature which is at least 10°C lower than the boiling temperature of the one or more apolar aromatic solvents, preferably at a temperature ranging between 85°C and a temperature which is at least 15°C lower than the boiling temperature of the one or more apolar aromatic solvents.

Optionally, a step of concentrating the solution comprising a mixture of neutral matters is carried out after step (c) and before step (d).

The process of the present disclosure can be carried out on any mixture of neutral matters from tall oil pitch. However, it was surprisingly discovered that the amount of the mixture of neutral matters obtained from tall oil pitch can be increased by extracting saponified tall oil pitch with one or more apolar aromatic solvent.

With preference, the one or more apolar aromatic solvents used in step (c) are selected among toluene and/or xylene; more preferably, the apolar aromatic solvent used in step (c) is toluene.

In a preferred embodiment, step (d) of distillation comprises one or more distillation sub-steps, so that step (d) of distillation comprises:

d1) performing a first distillation on the mixture of neutral matters, to obtain a first distillate fraction and a first bottom fraction;

d2) performing a second distillation on the first bottom fraction, to obtain a second distillate fraction and a second bottom fraction;

d3) optionally performing one or more further distillation on the second bottom fraction and further bottom fractions, to obtain a one or more further distillate fractions and one or more further bottom fractions;

wherein the distillate fraction that is dissolved in mixed solvents in step (e) is the second distillate fraction, or is a mixture of the second distillate fraction with the first distillate fraction and/or at least one further distillate fraction from the one or more distillate fractions.

With preference, wherein step (d) of distillation comprises one or more distillation sub-steps, the process is characterized in the one or more distillations sub-steps are one or more rectification steps, using a rectifying column.

With preference, the following parameters define the one or more rectification steps:

- The reflux ratio is comprised between 1/1 and 2/1.
- The rectification temperature is comprised between 240°C and 280°C, preferably between 245°C and 275°C.
- The rectifying column is coupled with a reflux condenser; with preference, the reflux condenser is at a temperature comprised between 110°C and 170°C.

With preference, one or more of the following embodiments can be used to better define the mixed solvents used in step (e):

- The mixed solvents used in step (e) comprise one or more apolar solvents selected from pentane, isopentane and/or hexane; with preference, the apolar solvent is or comprises hexane.
- The mixed solvents used in step (e) comprise one or more solvents having a ketone moiety selected from acetone and/or methyl ether ketone; with preference, the solvent having a ketone moiety is or comprises acetone.
- The mixed solvents used in step (e) comprise one or more monohydric alcohols selected from methanol, ethanol and/or propanol; with preference, the monohydric alcohol is or comprises methanol.

In a preferred embodiment, the mixed solvents comprise:

■ from 10 to 60 wt.% of one or more apolar solvents based on the total weight of the mixed solvents, preferentially from 15 to 55 wt.%, more preferentially from 20 to 50 wt.%, even more preferentially from 25% to 45 wt.%;
■ from 23 to 40 wt.% of one or more solvents having a ketone moiety based on the total weight of the mixed solvents, preferentially from 24 to 39 wt.%, more preferentially from 25 to 38 wt.%, even more preferentially from 26 to 37 wt.%;
■ from 0.5 to 2.5 wt.% of water based on the total weight of the mixed solvents, preferentially from 0.6 to 2.4 wt.%, more preferentially from 0.7 to 2.3 wt.%, even more preferentially from 0.8 to 2.2 wt.%; and
■ from 2 to 20 wt.% of one or more monohydric alcohols based on the total weight of the mixed solvents, preferentially from 3 to 18 wt.%, more preferentially from 4 to 17 wt.%, even more preferentially from 5 to 16 wt.%.

In a preferred embodiment, in step (e) the mass ratio between the mixed solvents and the distillate fraction is comprised between 1.0 and 2.0; preferably, the mass ratio is ranging from 1.1 to 1.7; more preferably is ranging from 1.2 to 1.5; even more preferably ranging from 1.2 to 1.4, or ranging from 1.2 to 1.3.

The one or more following embodiment can be used to better define step (f) of the process:

- The crystallization conditions of step (f) comprise a crystallization temperature ranging between 15°C and 25°C and/or a crystallization time of at least one hour, preferentially of at least two hours.
- Step (f) further comprises a step of performing an additional crystallization on the mother solution, to recover an additional extract of phytosterols and stanols, the additional crystallization being carried out under the same crystallization conditions of step (f); with preference, the additional extract of phytosterols and stanols is added to the extract of phytosterols and stanols recovered in step (f).
- The process further comprises an additional step of drying the extract of phytosterols and stanols, preferentially at a temperature of at least 100°C for at least 1 hour.
- Step (f) is carried out when the distillate fraction comprises a content less than or equal to 2.5 wt.% of $\alpha$-sitosterol based on the total weight of the distillate fraction and determined by gas chromatography with flame ionization detector analyses, preferably less than or equal to 2.4 wt.% of α-sitosterol, more preferably less than or equal to 1.7 wt.% of α-sitosterol.
- Step (f) is carried out when the distillate fraction comprises sterols in a content of at least 20 wt.% based on the total weight of the distillate fraction and determined by gas chromatography with flame ionization detector analyses, preferably at least 25 wt.%.

In a preferred embodiment, the process further comprises a step (g) of washing the extract of phytosterols and stanols with the mixed solvents used in step (f) at a temperature comprised between 10°C and 15°C, and/or an additional step of drying the extract of phytosterols and stanols, preferentially at a temperature of at least 100°C for at least 1 hour.

According to a second aspect, the disclosure provides an extract of phytosterols and stanols obtained from tall oil pitch, the extract being remarkable in that it comprises at least 65 wt.% of β-sitosterol based on the total weight of the extract of phytosterols and stanols, and less than 0.01 wt.% based on the total weight of the extract of phytosterols and stanols of each of oxysterols, α-sitosterol and betulin as determined by gas chromatography with flame ionization detector analyses.

## Description of the figures

- Figure 1 shows a chromatogram obtained on a sample before the step of crystallization.
- Figure 2 shows a chromatogram obtained on a sample after the step of crystallization.

## Detailed description of the disclosure

For the disclosure, the following definitions are given:
The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

The recitation of numerical ranges by endpoints includes all integer numbers and, where appropriate, fractions subsumed within that range (e.g. 1 to 5 can include 1, 2, 3, 4, 5 when referring to, for example, several elements, and can also include 1.5, 2, 2.75 and 3.80 when referring to, for example, measurements). The recitation of endpoints also includes the recited endpoint values themselves (e.g. from 1.0 to 5.0 includes both 1.0 and 5.0). Any numerical range recited herein is intended to include all sub-ranges subsumed therein.

The particular features, structures, characteristics or embodiments may be combined in any suitable manner, as would be apparent to a person skilled in the art from this disclosure, in one or more embodiments.

The disclosure provides a process for providing an extract of phytosterols and stanols from tall oil pitch, the process comprising the following steps:

a) providing tall oil pitch;
b) saponifying the tall oil pitch to produce saponified tall oil pitch by addition of a basic compound to the tall oil pitch;
c) extracting the saponified tall oil pitch with one or more apolar aromatic solvents, to produce a solution comprising a mixture of neutral matters;
d) performing a distillation of the mixture of neutral matters to obtain a distillate fraction and a bottom fraction;
e) dissolving the distillate fraction into mixed solvents, to obtain a dissolved distillate fraction,

the process is remarkable in that it further comprises a step (f) of crystallization which is performed on the dissolved distillate fraction under crystallization conditions to recover a mother solution and a crystallized fraction wherein the crystallized fraction forms an extract of phytosterols and stanols; and in that wherein the mixed solvents used on step (e) comprise one or more apolar solvents, one or more solvents having a ketone moiety, one or more monohydric alcohols and water.

## Production of a mixture of neutral matters from tall oil pitch

Tall oil pitch, which is obtained through the rectification of crude tall oil, is first saponified by mixing it with one or more basic components (such as NaOH, KOH or CsOH, preferably NaOH) dissolved in water while the mixture is heated, preferably refluxed. Thus, the temperature of the saponification step can be comprised between 100°C and 200°C, preferably between 110°C and 190°C, more preferably between 120°C and 180°C.

The reaction can occur in normal glassware, which is headed with a reflux condenser. Preferably, a Dean-Stark apparatus is used, since it allows the removal of the water from the reactor. The removal of water during the reaction contributes to the pull the thermodynamic equilibrium to the formation of the products, which is the saponified tall oil pitch.

The reaction time is at least one hour, preferably at least two hours, more preferably at least three hours. The reaction can be monitored by following the evolution of the water formation (and by taking into account the amount of water necessary to dissolve the basic component) that is removed during the reaction, and potentially collected thanks to the Dean-Stark apparatus.

Once the reaction is completed, the reaction medium is cooled down at room temperature and the saponified tall oil pitch is extracted. Any separatory funnel can be used. Preferably, a continuous extraction can be performed by using a Soxhlet apparatus.

The extraction temperature is preferably carried out at a temperature ranging between 80°C and a temperature which is at least 10°C lower than the boiling temperature of the extraction solvent. More preferably, the extraction temperature is comprised between 85°C and a temperature which is at least 15°C lower than the boiling temperature of the extraction solvent. The extraction temperature is at least 80°C and at most 270°C.

Aromatic apolar solvents, such as toluene and/or xylene, have been used and afford a yield of at least 45% of the mixture of neutral matters from tall oil pitch. Preferably, toluene is used.

Other solvents can be used, such as paraffinic solvents, cyclo-paraffinic solvent and/or terpenic hydrocarbon. Paraffinic solvents are preferably selected from hexane, heptane, octane, decane, dodecane, tridecane, undecane, petroleum ether (such as Nefras-C2) or their mixture. A cyclo-paraffinic solvent is preferably decalin. Terpenic hydrocarbon solvents are preferably selected from turpentine without pinene and/or hydrogenated terpenic hydrocarbons. These solvents afford a yield of at least 30% of the mixture of neutral matters from tall oil pitch and of at most 44% of the mixture of neutral matters from tall oil pitch.

A mixture of neutral matters of tall oil pitch comprises major phytosterols ($\beta$-sitosterol, campesterol) and stanols (sitostanol, campestanol). Up to 5% w/w $\alpha$-sitosterol (also named citrostandienol) are also present. They also comprise fatty alcohols, such as dehydro-4-abietol; pimara-7,15-dien-3-ol; behenyl alcohol and/or lignoceryl alcohol. Other components such as stigmastan-3,5-diene; $\Delta$5-avenasterols; 24-methylcycloartanol; methyl betulate; betulin and/or oxysterols can be found in the mixture of neutral matters of tall oil pitch.

### Separation of $\beta$-sitosterol through one or more rectifications

To provide an extract of phytosterols and stanols with a high content of $\beta$-sitosterol in high purity, it is necessary to purify the mixture of neutral matters.

The first step of this purification is performed through at least one distillation, preferentially at least one rectification.

Rectification is one implementation of distillation and its use includes fractionation of crude oil. If the distillate obtained during distillation is distilled again, a new distillate is obtained with an even higher concentration of volatile components. As the procedure is repeated, the concentration of volatile components in the distillate increases on each occasion. In practice, this multistage distillation process is carried out in the form of counter-current distillation (rectification) in a rectifying column. The rectifying column has an input line and an output line. The liquid mixture to be separated (*i.e.* the feed) is fed to the bottom of the column at the input line, where it is brought to boiling point. The vapour produced moves upwards inside the column exits it at the top (in the output line) and is condensed, through a reflux condenser. Part of the condensate is carried away as a top product. The remainder flows back into the column and moves downwards as the liquid opposite phase.

The rectification temperature, namely inside the rectification column, is advantageously comprised between 240°C and 280°C, preferably between 245°C and 275°C, more preferably between 250°C and 270°C, even more preferably between 255°C and 265°C.

The temperature at the bottom of the column is advantageously comprised between 250°C and 285°C, preferably between 255°C and 280°C, more preferably between 260°C and 275°C, even more preferably between 255°C and 270°C.

The temperature of the reflux condenser is advantageously comprised between 110°C and 170°C, preferably between 115°C and 165°C, more preferably between 120°C and 160°C, even more preferably between 125°C and 155°C.

The temperature of the condensate is advantageously comprised between 150°C and 210°C, preferably between 155°C and 205°C, more preferably between 160°C and 200°C, even more preferably between 165°C and 195°C.

The pressure at the top of the column is advantageously comprised between 0.05 mbar and 0.35 mbar.

The pressure at the bottom of the column is advantageously comprised between 2 mbar and 6 mbar.

Generally, to increase the amount of the major sterols into the distillate fraction, the bottom fraction resulting after a first rectification is processed to another step of distillation or rectification. In this way, it is possible to obtain a distillate fraction comprising between 25 and 55 wt.% of sterols based on the total weight of the distillate fraction. The sterol recovery rate from the neutral matter of tall oil pitch in the volatile distillate fraction is at least equal to 80%. Such distillate fraction also usually comprises between 0.5 and 1.7 wt.% of α-sitosterol based on the total weight of the distillate fraction. In the same time, α-sitosterol is accumulated in the non-volatile bottom fraction in a concentration ranging between 1 and 4 wt.% based on the total weight of the non-volatile bottom fraction, while other sterols in the non-volatile bottom fraction show a concentration between 7 and 30 wt.% based on the total weight of the non-volatile bottom fraction.

## Removal of impurities by crystallization of β-sitosterol

The rectification process provides a distillate fraction comprising major sterols (campesterol and β-sitosterol) in an interesting amount but also comprising impurities such as α-sitosterol, oxysterols and betulin.

A crystallization process is thus undertaken to remove the above-mentioned impurities.

Advantageously, the crystallization is carried out when the distillate fraction comprises a low amount of α-sitosterol. Such low amount can be less than or equal to 2.5 wt.% of α-sitosterol based on the total weight of the distillate fraction and determined by gas chromatography-flame ionization α-sitosterol detector (GC-FID) analyses, preferably less than 2.4 wt.% of α-sitosterol., more preferably less than 2.0 wt.% of α-sitosterol, even more preferably less than 1.7 wt.% of α-sitosterol.

It is also advantageous that the crystallization is carried out when the distillate fraction comprises a high quantity of sterols. Such high quantity can be at least 20 wt.% of sterols based on the total weight of the distillate fraction and determined by gas chromatography-flame ionization detector (GC-FID) analyses, preferably at least 21 wt.%, more preferably at least 22 wt.%, even more preferably at least 25 wt.%.

It is further advantageous that the crystallization is carried out when the distillate fraction comprises a low amount of α-sitosterol such as being less than or equal to 2 wt.%; and a high quantity of sterols, such as being at least 20 wt.%, wherein the weight percentages are based on the total weight of the distillate fraction and determined by gas chromatography-flame ionization detector (GC-FID) analyses.

The distillate fraction is firstly dissolved into mixed solvents being a mixture of at least four different types of solvent, and comprising

- one or more apolar solvents;
- one or more solvents having a ketone moiety,
- one or more monohydric alcohols, and
- water.

Each solvent of a kind is added for a specific purpose and the mixed solvent has a dissolving power that is different than each of the components of the mixture.

Apolar solvents and paraffinic hydrocarbons, in particular, show a high dissolving ability on di- and triterpenic hydrocarbons and condensed products (resins) that appear from tall oil pitch. The longer is the chain of paraffinic hydrocarbon, the higher is its dissolving ability. The apolar solvents can be selected from pentane, isopentane, and/or hexane, preferably hexane which is the best choice due to its adequate boiling point.

Monohydric alcohols are excellent solvents for sterols, due to their chemical affinity. Water can be added to adjust the dissolving ability of an alcohol. The one or more monohydric alcohols can be selected from methanol, ethanol, and/or propanol, preferably methanol.

The solvent having a ketone moiety is acetone and/or methyl ether ketone, preferentially acetone. (Ketones is used to separate α-sitosterol since they increase the solubility of α-sitosterol.

Furthermore, by adding water, sterols can be recrystallized as explained in US 6,465,665.

The mixed solvents advantageously comprise between 10 wt.% and 60 wt.% of apolar solvent, between 23 wt.% and 40 wt.% of solvent having a ketone moiety, between 0.5 wt.% and 2.5 wt.% of water and between 23 wt.% and 40 wt.% of one or more monohydric alcohols based on the total weight of the mixed solvents.

The mass ratio between the mixed solvents and the distillate fraction is comprised between 1.00 and 2.00, with preference is the mass ratio is 1.25.

During the crystallization, the whole distillate fraction must be dissolved into the mixed solvents. Heating and/or stirring the solution increases the dissolution capacity of the solvent. The temperature can thus be increased to a dissolving temperature comprised between 35°C and 60°C, preferentially between 40°C and 55°C.

Once the whole distillate fraction has been dissolved, the stirring is stopped and the solution is cooled down to a temperature comprised between 15°C and 25°C, preferentially to 20°C.

The crystallization process is thus started and last for at least one hour, preferably at least two hours. It is noteworthy to mention that a crystallization temperature at room temperature (between 15°C and 25°C) conducted in the presence of the mixed solvents has allowed for obtaining an extract of phytosterols and stanols which is free of triterpene alcohols and betulin in particular. It is a surprising result since it is found from WO 2019/050430 that temperature lower than 50°C usually leads to the co-crystallization of betulin and phytosterols.

Once all the crystals have been formed from the mother liquor, they can be recovered through filtration. Optionally, the crystals can be washed with the mixed solvents at a temperature comprised between 5°C and 10°C.

The mother liquor can also be recovered. After evaporation of between 25% and 50% of its volume, at rotary film evaporator, the concentrated portion of the mother liquor is cooled down to a temperature comprised between 15°C and 25°C, preferentially to 20°C, to start a new crystallization process. The crystals can then be collected and can optionally be washed with the mixed solvents at a temperature comprised between 5°C and 10°C.

Afterwards, both portions of the crystals can be dried, for example in an oven or a curing chamber, preferentially at a temperature of at least 100°C, more preferentially of at least 110°C. The drying process can preferentially last for at least 1 hour, more preferentially for at least 2 hours. They can also be dried under reduced pressure (i.e., under vacuum) to accelerate the drying process.

The low temperature (between 5°C and 10°C) used for washing the crystals prevents that the collected crystals are redissolved. However, since it is not possible to avoid completely any redissolution, a purification of the filtrates can be undertaken. Both filtrates can thus be mixed and cooled down to a temperature comprised between 15°C and 25°C, preferentially to 20°C, to start a new crystallization process.

This way to proceed allows for the recovery of a maximum of an extract of phytosterols and stanols. The extract of phytosterols and stanols is crystalline, comprises an elevated amount of β-sitosterols and is devoid of impurities such as α-sitosterol, but also oxysterols and betulin.

## Test and determination methods

In the techniques of distillation (also including the rectification), the reflux is the liquid condensed from the rising vapour which returns to the pot flask. From this, the "reflux ratio" is the ratio between the boil-up rate and the take-off rate. In other words, it is the ratio between the amount of reflux that goes back down the distillation column and the amount of reflux that is collected in the receiver (as distillate). Thus, if 5 parts of the reflux go back down the distillation column and 1 part is collected as distillate, then the reflux ratio is 5/1. In the case where all the reflux is collected as distillate, the reflux ratio would be zero. If no distillate is collected, then the reflux ratio is not assigned. Instead, it is called "total reflux" or "equilibration". The higher the reflux ratio, the more vapour/liquid contact can occur in the distillation column and the collection rate for the distillate will be slower.

To determine the content of the extract or of the crystal product obtained by the process of the present disclosure, gas chromatography-mass spectrometry (GC-MS) and/or gas chromatography with flame ionization detector (GC-FID) analyses were used.

GC-MS has been used for the identification and quantification of the components. The apparatus is Agilent 7890B-GC/5977A-MS. The column is 5%-phenyl-95%-polymethylsiloxane phase (HP-5ms 60m x 0.125mm x 0.125 μm). The temperature program is as followed: start 120°C/5 min, heating 5°C/min, isotherm 350°C/10 min. The injection parameters are 1 μl, at 350°C with a split of 1/10. The mobile phase is helium 99.9995%. The transfer line has a temperature of 360°C. The MSq program is TIC, scan mode, 28-800Da, 70 eV. Mass spectral library NIST-11 was used. The limit of detection of this analytical method is 0.01 % w/w.

GC-FID has been used for routine analysis. The apparatus is Chromatec-5000. The column is polymethylsiloxane phase (SE-30 30m x 0.125mm x 0.125 μm). The temperature program is as followed: start 50°C/5 min, heating 5°C/min, isotherm 350°C/10 min. The injection parameters are 1 μl, at 350°C with a split of 1/10. The mobile phase is nitrogen. The limit of detection of this analytical method is also 0.01 % w/w.

Karl Fischer titration is an analytical method that uses colorimetric or volumetric titration to determine trace amounts of water in a sample.

The sterol recovery rate in example 1 has been determined according to formula (1):

$$Sterol\ recovery\ rate = \frac{sterols\ in\ the\ extract}{[sterols\ in\ pitch]*(1-water\ content-alkali\ content)} * yield \qquad (1)$$

Similarly, in other examples, the sterol recovery rate has been determined according to formula (2):

$$Sterol\ recovery\ rate = \frac{sterols\ in\ the\ sample}{sterol\ in\ the\ crude\ material} * yield \qquad (2)$$

Examples

The embodiments of the present disclosure will be better understood by looking at the example below.

**Example 1: Production of a mixture of neutral matters from tall oil pitch**

Saponification of tall oil pitch

A reactor of non-corrosive material with an overhead paddle stirrer, heated through jacket with Dean-Stark head, is loaded with 2500 g of tall oil pitch containing 9.3 % w/w of the total of six major transformable sterols (as determined by GC-MS experiments) and added 375 g of sodium hydroxide dissolved in 500 g of water. Saponification occurred at 130-135°C for 3 hours using a Dean-Stark head to separate water evolving during the reaction. It should be noted that there was a considerable increase in the volume of the reaction mix during saponification. At the end of the process, the reaction mix is drained through the bottom into an aluminium mould; 2800 g of the solid saponified pitch have been formed instantaneously. Fisher titration method has allowed determining that there is an alkali content of 7.5% and a water content of 8.1% in the solid saponified pitch, as based on the total weight of the solid saponified pitch). The melting temperature has been determined to be of 117.1°C. At the beginning of the process the water has been condensing through Dean-Stark head, while after 1.5 hours, water pick-up ended. In total, 40 g of water have been picked-up through the head.

Extraction of saponified tall oil pitch

The extraction of saponified tall oil pitch is performed in a 150 ml glass Soxhlet extractor. Small, 2-3 mm pieces of solid saponified tall oil pitch were put into a paper cartridge placed into the extractor. The bottom flask was filled with such amount of solvent as twice of Soxhlet extractor capacity and heated till boiling with a reflux condenser for 2 hours. The solvent was skimmed from this extraction solution with a rotary film evaporator.

Table 1 reports the extraction parameters and the composition of extracts.

Table 1: Extraction of saponified tall oil pitch: conditions and results.

| | solvent | hexane (b.p. = 68°C) | nefras-C2 (b.p. = 120°C) | toluene (b.p. = 110°C) |
|---|---|---|---|---|
| saponified tall oil pitch to solvent ratio | | 1:15 | 1:12 | 1:10 |
| Extraction time (hours) | | 2 | 2 | 2 |

| Extraction temperature (°C) | 80 | 110 | 100 |
|---|---|---|---|
| Extraction yield (%) | 25.7 | 19.3 | 48.6 |
| **Composition of extract#** | | | |
| Total amount of fatty alcohols (wt.%) | 3.8 | 3.9 | 1.8 |
| **Total amount of sterols (wt.%)** | **11.7** | **13.1** | **8.3** |
| Total of detected components (wt.%) | 61.9 | 54 | 46.2 |
| **Sterol recovery rate in the neutral matter from tall oil pitch (%)** | **38.4** | **32.2** | **52.9** |

# determined by GC-FID experiments.

Using toluene as extraction solvent allows for obtaining a higher sterol recovery rate in the mixture of neutral matters, when tall oil pitch is used as starting materials and treated through a saponification step, an extraction step and concentration. By comparison with known results described in example 1 of US 6,465,665, the sterol recovery rate is 11.1% higher when toluene is used as the extraction solvent.

**Example 2: Separation of β-sitosterol through one or more rectifications**

A mixture of neutral matters obtained from tall oil pitch is provided. Such a mixture can comprise up to 5 wt.% of α-sitosterol based on the total weight of the mixture of neutral matters. Such a mixture of neutral matters can be either the one of example 1 (see its composition in table 1) or another one. For example 2, the composition of the mixture of neutral matters is given in table 2 under the column entitled "Material in the feed".

The mixture is continuously fed into a glass rectification column with an inner diameter of 50 mm and a height of 450 mm. The glass rectification column is also fitted with electric heating to compensate for heat loss, one reflux condenser and one phlegm separator on top. A non-volatile product receiver, with a volume of 1 litre, as well as a vacuum system is also provided.

First batch

A first batch comprising a mixture of neutral matters obtained from tall oil pitch is fed into the rectification column. Upon the rectification, the first distillate cut contained a low amount of major sterols (7.2 wt.%), as the sterols remain concentrated mainly in the non-volatile bottom fraction (26.2 wt.%). The two major sterols that are detected are campesterol and β-sitosterol. The presence of α-sitosterol was not detected in the distillate fraction of the first rectification. The results and the conditions of the first rectification are visible in table 2.

Table 2: First rectification of a first batch comprising a mixture of neutral matters obtained from tall oil pitch.

| RECTIFICATION 1 (first batch) | | | |
|---|---|---|---|
| | Material in the feed (wt. %) | Distillate (wt.%) | Bottom (wt. %) | Conditions#<br><br>Flow = 400 ml/h |
| total of fatty alcohol | 6.0 | 27.3 | 0.4 | **Temperature** (°C) |

(continued)

| RECTIFICATION 1 (first batch) | | | | |
|---|---|---|---|---|
| | Material in the feed (wt. %) | Distillate (wt.%) | Bottom (wt. %) | Conditions[#] |
| sitostanol | 3.5 | 0.6 | 4.5 | - rectification = 250<br>- bottom of the column = 260<br>- condensate = 160<br>- condenser = 120 |
| campestanol | 1.6 | 1.3 | 1.4 | |
| **campesterol** | 4.2 | 3.2 | 4.1 | **Vacuum** (mbar) |
| β-**sitosterol** | 18.4 | 4 | 22.1 | - top of the column = 0.09<br>- bottom of the column = 2.8 |
| **Total of sterols** | **22.6** | **7.2** | **26.2** | **Reflux ratio** = 2/1 |
| Oxysterols | 5.0 | n.d. | n.d. | |
| α-**sitosterol** | 1.7 | **n.d.** | 2.1 | [#] GC-FID analyses have been carried out to determine the composition of the feed, of the distillate fraction and of the bottom fraction. |
| betulin | 2.0 | n.d. | 1.9 | |
| heavy components | 18.4 | 1.6 | 29.6 | |
| n.d. = not detected;<br>the yield of the rectification for the volatile distillate fraction is 17% and for the non-volatile bottom fraction is 81.6%. | | | | |

The non-volatile bottom fraction (see its composition on table 2) is processed through another rectification.

By comparison with the first rectification, the temperature of the rectifying column has been increased by 10°C (from 250°C to 260°C). The temperature of the reflux condenser has also been increased (from 120°C to 160°C). The reflux ratio of the rectifying column has decreased from 2/1 to 1/1, meaning that the collection rate of the distillate has increased.

This allows to obtain a distillate fraction with a higher content of major sterols (44.3 wt.%) and to detect a small amount (1.2 wt.%) of α-sitosterol.

The results and the conditions of the second rectification are visible on table 3.

Table 3: Second rectification applied on the bottom fraction of the first rectification.

| RECTIFICATION 2 (first batch) | | | | |
|---|---|---|---|---|
| | Material in the feed (wt. %) | Distillate (wt.%) | Bottom (wt. %) | Conditions[#] |
| total of fatty alcohol | 0.4 | 0.05 | n.d. | **Flow** = 400 ml/h<br>**Temperature** (°C) |
| sitostanol | 4.5 | 7.5 | 4.2 | - rectification = 260<br>- bottom of the column = 265<br>- condensate = 200<br>- **condenser = 160** |
| campestanol | 1.4 | 2.8 | 0.5 | |
| **campesterol** | 4.1 | 7.6 | 1.9 | **Vacuum** (mbar) |
| β-**sitosterol** | 22.1 | 36.7 | 18 | - top of the column = 0.06<br>- bottom of the column = 4 |
| **Total of sterols** | **26.2** | **44.3** | **19.9** | **Reflux ratio** = 1/1 |
| Oxysterols | n.d. | 7.7 | 8.9 | |

(continued)

| RECTIFICATION 2 (first batch) | | | | |
|---|---|---|---|---|
| | Material in the feed (wt. %) | Distillate (wt.%) | Bottom (wt. %) | Conditions# |
| α-sitosterol | 2.1 | **1.2** | 2.8 | # GC-FID analyses have been carried out to determine the composition of the feed, of the distillate fraction and of the bottom fraction. |
| betulin | 1.9 | n.d. | 1.5 | |
| heavy components | 29.6 | 36.4 | 32.1 | |
| n.d. = not detected; | | | | |

The yield of the rectification for the volatile distillate fraction is 35% and for the non-volatile bottom fraction is 65%.

The non-volatile bottom fraction (see its composition on table 3) is processed through another rectification.

By comparison with the second rectification, the temperature of the reflux condenser has been decreased by 10°C (from 160°C to 150°C).

A small amount (1.6 wt.%) of α-sitosterol has also been detected. The results and the conditions of the third rectification are visible on table 4.

Table 4: Third rectification applied on the bottom fraction of the second rectification.

| RECTIFICATION 3 (first batch) | | | | |
|---|---|---|---|---|
| | Material in the feed (wt. %) | Distillate (wt.%) | Bottom (wt. %) | Conditions# |
| | | | | **Flow** = 400 ml/h |
| total of fatty alcohol | n.d. | n.d. | n.d. | **Temperature** (°C) |
| sitostanol | 4.2 | 7.3 | 3.2 | - rectification = 260<br>- bottom of the column = 265 |
| campestanol | 0.5 | 1.7 | 0.3 | - condensate = 200<br>- condenser = 150 |
| **campesterol** | 1.9 | 5 | 1 | **Vacuum** (mbar) |
| β-**sitosterol** | 18 | 32.5 | 12.3 | - top of the column = 0.06<br>- bottom of the column = 4 |
| **Total of sterols** | **19.9** | **37.5** | **13.3** | **Reflux ratio** = 1/1 |
| Oxysterols | 8.9 | 8 | 9.3 | |
| α-**sitosterol** | 2.8 | **1.6** | 3.3 | # GC-FID analyses have been carried out to determine the composition of the feed, of the distillate fraction and of the bottom fraction. |
| betulin | 1.5 | 0.1 | 2.2 | |
| heavy components | 32.1 | 38.9 | 37 | |
| n.d. = not detected;<br>the yield of the rectification for the volatile distillate fraction is 35% and for the non-volatile bottom fraction is 65%. | | | | |

It is notable that in this case, namely after having performed three rectification steps, the volatile distillate fraction does not contain a detectable amount of fatty alcohol.

Second batch

Table 5 reports the results of the rectification of a second batch of neutral substances obtained from tall oil pitch, using different rectification conditions.

Table 5: Rectification of a second batch of neutral substances obtained from tall oil pitch.

| RECTIFICATION ON A SECOND BATCH | | | | |
|---|---|---|---|---|
| | Material in the feed (wt. %) | Distillate (wt.%) | Bottom (wt. %) | Conditions[#]<br><br>**Flow** = 250 ml/h |
| total of fatty alcohol | 5.1 | 7 | n.d. | **Temperature** (°C) |
| sitostanol | 3.6 | 4.4 | 2.1 | - rectification = 260 |
| campestanol | 1.8 | 2.6 | 0.3 | - bottom of the column = 275<br>- condensate = 200 |
| **campesterol** | 3.4 | 4.7 | 0.8 | - condenser = 140<br><br>**Vacuum** (mbar) |
| $\beta$-**sitosterol** | 18.2 | 21.8 | 8.7 | - top of the column = 0.3 |
| **Total of sterols** | **21.6** | **26.5** | **9.5** | - bottom of the column = 5<br><br>**Reflux ratio** = 1/1 |
| Oxysterols | 4.8 | 5.8 | 8.6 | |
| $\alpha$-**sitosterol** | 1.3 | 0.7 | 1.9 | |
| betulin | 1.1 | n.d. | 3.7 | [#] GC-FID analyses have been carried out to determine the composition of the feed, of the distillate fraction and of the bottom fraction. |
| heavy components | 16.4 | 16.8 | 16.5 | |
| n.d. = not detected;<br>the yield of the rectification for the volatile distillate fraction is 66% and for the non-volatile bottom fraction is 34%; | | | | |

The sterol recovery rate from the neutral matter of tall oil pitch in the volatile distillate fraction, calculated according to formula (2), is at least equal to 80.97%.

Upon the rectification conditions described in table 5, the distillate cut contained an elevated amount of major sterols (26.5 wt.%). The presence of $\alpha$-sitosterol was detected in the distillate fraction at an amount of 0.7 wt.%.

**Example 3: Removal of impurities by crystallization of $\beta$-sitosterol**

**Example 3A**

The volatile distillate fraction recovered from the second batch (see its composition on table 5) has been further treated as follows:

A glass reactor, fitted with a paddle stirrer, a heating jacket, a reflux condenser and 1000 ml metering apparatus, was loaded with 200 g of source material and 248.0 g of mixed solvents comprising hexane, monohydric alcohol, water and acetone, to form a mixture. The hexane is present in 45 wt.%, the monohydric alcohol is methanol and is present in an amount of 16 wt.%, the water is present in 2 wt.% and the acetone is present in 37 wt.%. Then the mixture between the mixed solvents and the source material was stirred and heated to 45°C through reactor jacket. Stirring continued till complete dissolution of the content. Afterwards, heating and stirring were stopped and the mother solution was cooled down to 20°C. The solution was maintained for another 2 hours at 20°C to let $\beta$-sitosterol crystallize. The process of crystallization results in large crystals of $\beta$-sitosterol that can be carefully decanted from the mother solution. Once the mother solution has been separated, the crystalline mass was put on Büchner funnel with Bunsen flask to remove the remaining mother solution with a vacuum. $\beta$-sitosterol crystals were washed right on the filter with 150 g of mixed solvents at a temperature of 6°C, and the filtrate was collected.

The mother solution was put into the flask of a rotary film evaporator, then 1/3 of the solvent volume was removed. The remaining portion was left for crystallization of further crystals of $\beta$-sitosterol under the conditions as described above. The same procedure as above was used to separate crystals. In the end, $\beta$-sitosterol crystals were washed on the Büchner funnel (or on the filter) with 150 g of mixed solvents at a temperature of 6°C, and the filtrate was collected.

Once both filtrates were collected, they were mixed and additional β-sitosterol crystals were recrystallized from them under conditions as described above.

At this stage, the mother liquor of this crystallization was collected, concentrated at rotary film evaporator (temperature of the bath at 120°C, a vacuum of 10 mbar) to obtain a first barren residue.

The additional β-sitosterol crystals were collected and washed with 25 g of mixed solvents at a temperature of 6°C. The washing solution was recovered and concentrated at rotary film evaporator (temperature of the bath at 120°C, a vacuum of 10 mbar) to obtain a second barren residue.

Table 6 reports the composition of the first and second barren residue.

Table 6: Composition of the first and second barren residue from the crystallization of distillate cuts after rectification of the second batch.

| Component | First barren residue (wt.%)[#] | Second barren residue (wt.%)[#] |
|---|---|---|
| total of fatty alcohol | 6.2 | 27.0 |
| sitostanol | 2.4 | 7.1 |
| campestanol | 3.3 | 1.5 |
| **campesterol** | 4.1 | 7.5 |
| **β-sitosterol** | 9.9 | 34.4 |
| **Total of sterols** | **14** | **41.9** |
| Oxysterols | 8.7 | 1.3 |
| **α-sitosterol** | 1.0 | 0.2 |
| betulin | n.d. | n.d. |
| heavy components | 26.7 | 5.0 |
| n.d. = not detected; [#] determined by GC-FID experiments. | | |

The yield for the 1st barren residue has been determined to be 71% and the yield for the 2nd barren residue has been determined to be 10.15%.

The sterol recovery rate calculated according to formula (2) in the first barren residue and in the second barren residue is respectively equal to 37.51% and 16.05%.

The barren residues comprise a detectable quantity of oxysterols and α-sitosterol. The quantity of these impurities is logically considerably inferior in the second barren residue obtained from the washing solution than in the first barren residue, obtained from the concentration of the mother liquor made of two filtrates.

All the β-sitosterol crystals were put together and dried from remaining solvent in a curing chamber at 115°C for two hours. Table 7 reports the composition of the β-sitosterol crystals that have been obtained.

Table 7: Crystallization of distillate cuts after rectification of the second batch.

| Component | Crystal product (wt.%)[#] |
|---|---|
| total of fatty alcohol | 1.8 |
| **sitostanol** | **12.1** |
| **campestanol** | **1.7** |
| **campesterol** | **14.2** |
| **β-sitosterol** | **67.0** |
| Oxysterols | n.d. |
| α-sitosterol | n.d. |
| betulin | n.d. |
| heavy components | 1.4 |
| **Total of detected component** | **98.2** |

(continued)

| Component | Crystal product (wt.%)# |
|---|---|
| **Total of sterols and stanols** | **95.0** |

n.d. = not detected;
# determined by GC-FID experiments;
The sterol recovery rate calculated according to formula (2) is equal to 56.38%.

The crystallization yield has been determined to be 18.4% and the total amount of detected component is 98.2 wt.%, as indicated in table 7. The method of the present disclosure allows for obtaining 95 wt.% of sterols and stanols with only 1.8 wt.% of fatty alcohols (wax).

Thanks to the present method (rectification of neutral matter of tall oil pitch followed by crystallization of β-sitosterol from the distillate fraction), the end product is a crystal that mainly comprises β-sitosterol and which is free of oxysterols, betulin and α-sitosterol. Moreover, the sterol recovery rate for the crystallization procedure is at least 55%.

The sterol recovery rate from the neutral matter of tall oil pitch, namely from the starting material used in the process of the present disclosure, is at least 45%.

**Example 3B**

A third batch similar to the first batch after two rectifications (similar to the data given on table 3) has been provided.

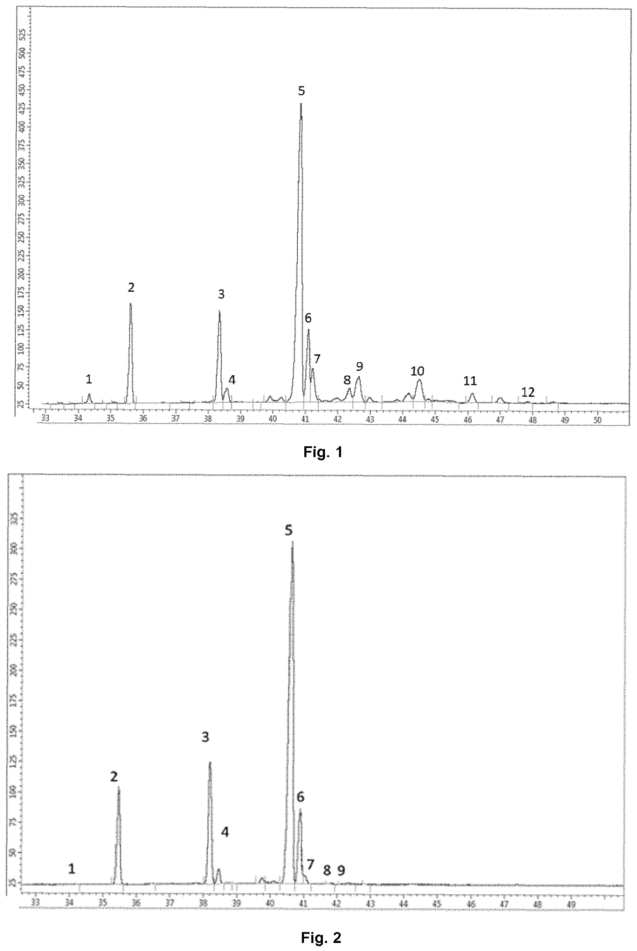

Table 8 reports the content of the distillate fraction, before being recrystallized as well as the detailed analysis of the chromatogram of figure 1.

Table 8: Composition of the distillate fraction before crystallization as well as the assignment of the peak numbers and the retention time of figure 1.

| Composition of the third batch (wt.%)# | | Peak number§ in figure 1 | Retention time (min) |
|---|---|---|---|
| total of fatty alcohol | 0.36 | - | - |
| **sitostanol** | **7.6** | 6 | 41.085 |
| **campestanol** | **1.8** | 4 | 38.592 |
| **campesterol** | **9.2** | 3 | 38.340 |
| **β-sitosterol** | **45.1** | 5 | 40.630 |
| Oxysterols | 4.3 | 10 | 44.509 |
| α-sitosterol | 1.3 | 11 | 46.145 |
| betulin | 0.2 | 12 | 47.859 |
| stigmastan-3,5-diene | 1.0 | 1 | 34.331 |
| Δ5-avenasterol | 3.6 | 7 | 41.213 |
| methyl betulate | 4.3 | 8 | 42.361 |
| 24-methylcycloartanol | 3.2 | 9 | 42.645 |
| Total of detected components | 81.96 | - | - |

§ Peak number 2 is the peak of the internal standard, cholesterol (35.599 min)
# determined by GC-FID experiments.

β-sitosterol crystals were obtained from the third batch, as described in example 3A.

Table 9 reports the composition of the β-sitosterol crystals that have been obtained.

Table 9: Crystallization of distillate cuts after rectification as well as the assignment of the peak number and the retention time of figure 2 for the third batch

| Component | Crystal product (wt. %)# | Peak number§ in figure 2 | Retention time (min) |
|---|---|---|---|
| **sitostanol** | **11.5** | 6 | 40.884 |
| **campestanol** | **2.0** | 4 | 38.467 |
| **campesterol** | **17.7** | 3 | 38.190 |
| **β-sitosterol** | **65.1** | 5 | 40.629 |
| Oxysterols | n.d. | n.d. | n.d. |
| α-sitosterol | n.d. | n.d. | n.d. |
| betulin | n.d. | n.d. | n.d. |
| stigmastan-3,5-diene | 0.04 | 1 | 34.229 |
| Δ5-avenasterol | 1.2 | 7 | 41.032 |
| methyl betulate | 0.2 | 8 | 41.846 |
| 24-methylcycloartanol | 0.3 | 9 | 42.360 |
| Total of detected components | 98.04 | - | - |
| **Total of sterols and stanols** | **96.30** | - | - |

n.d. = not detected
§ Peak number 2 is the peak of the internal standard, cholesterol (35.456 min)
#determined by GC-FID experiments.

The total amount of the detected component is 98.04 wt.%, as indicated in table 9. The method of the present disclosure allows for obtaining 96.30 wt.% of sterols and stanols without any traces of fatty alcohols (wax).

As it can be seen from the comparison of figures 1 and 2, the peaks numbered 10, 11 and 12 have completely disappeared on the chromatogram taken after the crystallization of the sample. Therefore, it is concluded that the extract of phytosterols and stanols obtained according to the present method comprises less than 0.01 wt.% based on the total weight of the extract of phytosterols and stanols of each of oxysterols, betulin and α-sitosterol.

**Claims**

1.   Process for providing an extract of phytosterols and stanols from tall oil pitch, the process comprising the following steps:

a) providing a tall oil pitch;
b) saponifying the tall oil pitch to produce a saponified tall oil pitch by addition of a basic compound to the tall oil pitch:
c) extracting the saponified tall oil pitch with one or more apolar aromatic solvents, to produce a solution comprising a mixture of neutral matters;
d) performing a distillation of the mixture of neutral matters to obtain a distillate fraction and a bottom fraction;
e) dissolving the distillate fraction into mixed solvents, to obtain a dissolved distillate fraction,

the process is **characterized in that** it further comprises a step (f) of crystallization which is performed on the dissolved distillate fraction under crystallization conditions to recover a mother solution and a crystallized fraction, wherein the crystallized fraction forms an extract of phytosterols and stanols; and **in that** the mixed solvents used on step (e) comprise one or more apolar solvents, one or more solvents having a ketone moiety, one or more monohydric alcohols and water.

2.   The process according to claim 1, **characterized in that** step (d) of distillation comprises one or more distillation sub-steps so that step (d) of distillation comprises:

d1) performing a first distillation on the mixture of neutral matters, to obtain a first distillate fraction and a first bottom fraction;

d2) performing a second distillation on the first bottom fraction, to obtain a second distillate fraction and a second bottom fraction;

d3) optionally performing one or more further distillation on the second bottom fraction and further bottom fractions, to obtain a one or more further distillate fractions and one or more further bottom fractions;

wherein the distillate fraction that is dissolved in mixed solvents in step e) is the second distillate fraction, or is a mixture of the second distillate fraction with the first distillate fraction and/or at least one further distillate fraction from the one or more distillate fractions.

3. The process according to any one of claim 1 or 2, **characterized in that** the mixed solvents used in step (e) comprise one or more apolar solvents selected from pentane, isopentane and/or hexane; with preference, the apolar solvent is or comprises hexane.

4. The process according to claims 1 or 3, **characterized in that** the mixed solvents used in step (e) comprise one or more solvents having a ketone moiety selected from acetone and/or methyl ether ketone; with preference, the solvent having a ketone moiety is or comprises acetone.

5. The process according to any one of claims 1 to 4, **characterized in that** the mixed solvents used in step (e) comprise one or more monohydric alcohols selected from methanol, ethanol and/or propanol; with preference, the monohydric alcohol is or comprises methanol.

6. The process according to any one of claims 1 to 5, **characterized in that** the mixed solvents used in step (e) comprise:

- from 10 to 60 wt.% of one or more apolar solvent based on the total weight of the mixed solvents;
- from 23 to 40 wt.% of one or more solvent having a ketone moiety based on the total weight of the mixed solvents;
- from 0.5 to 2.5 wt.% of water based on the total weight of the mixed solvents, and
- from 2 to 20 wt.% of one or more monohydric alcohols based on the total weight of the mixed solvents.

7. The process according to any one of claims 1 to 6, **characterized in that**, in step (e) the mass ratio between the mixed solvents and the distillate fraction is comprised between 1.0 and 2.0; preferably, the mass ratio is ranging from 1.1 to 1.7; more preferably is ranging from 1.2 to 1.5.

8. The process according to any one of claims 1 to 7, **characterized in that** the crystallization conditions of step (f) comprise a crystallization temperature ranging between 15°C and 25°C and/or a crystallization time of at least one hour.

9. The process according to any one of claims 1 to 8, **characterized in that** step (f) further comprises a step of performing an additional crystallization on the mother solution, to recover an additional extract of phytosterols and stanols, the additional crystallization being carried out under the same crystallization conditions of step (f); with preference, the additional extract of phytosterols and stanols is added to the extract of phytosterols and stanols recovered in step (f).

10. The process according to any one of claims 1 to 9, **characterized in that** the process further comprises

- a step (g) of washing the extract of phytosterols and stanols with the mixed solvents used in step (f) at a temperature comprised between 10°C and 15°C; and/or
- an additional step of drying the extract of phytosterols and stanols, preferentially at a temperature of at least 100°C for at least 1 hour.

11. The process according to any one of claims 1 to 10, **characterized in that** step (f) is carried out when the distillate fraction comprises

- a content less than or equal to 2.5 wt.% of $\alpha$-sitosterol based on the total weight of the distillate fraction and determined by gas chromatography with flame ionization detector analyses, preferably less than or equal to 1.7 wt.% of α-sitosterol; and/or
- a content of at least 20 wt.% of sterols based on the total weight of the distillate fraction and determined by gas chromatography with flame ionization detector analyses, preferably at least 25 wt.%.

12. The process according to any one of claims 1 to 11, **characterized in that** step (c) of extracting the saponified tall oil pitch with one or more apolar aromatic solvents is carried out at a temperature ranging between 80°C and a temperature which is at least 10°C lower than the boiling temperature of the one or more apolar aromatic solvents.

13. The process according to any one of claims 1 to 12, **characterized in that** the one or more apolar aromatic solvents used in step (c) are selected among toluene and/or xylene; preferably, the apolar aromatic solvent used in step (c) is toluene.

14. The process according to any one of claims 2 to 13, wherein step (d) of distillation comprises one or more distillations sub-steps, the process is **characterized in** the one or more distillations sub-steps are one or more rectification steps, using a rectifying column; with preference, the following parameters defining the one or more rectification steps

- the reflux ratio is comprised between 1/1 and 2/1; and/or
- the rectification temperature is comprised between 240°C and 280°C, and/or
- the rectifying column is coupled with a reflux condenser; with preference, the reflux condenser is at a temperature comprised between 110°C and 170°C.

15. Extract of phytosterols and stanols obtained from tall oil pitch, the extract being **characterized in that** it comprises at least 65 wt.% of β-sitosterol based on the total weight of the extract of phytosterols and stanols, and less than 0.01 wt.% based on the total weight of the extract of phytosterols and stanols of each of oxysterols, α-sitosterol and betulin as determined by gas chromatography with flame ionization detector analyses.

**Patentansprüche**

1. Verfahren zur Bereitstellung eines Extrakts von Phytosterolen und Stanolen aus Tallölpech, wobei das Verfahren die folgenden Schritte umfasst:

a) Bereitstellen eines Tallölpechs;
b) Verseifen des Tallölpechs unter Bildung eines verseiften Tallölpechs durch Zugabe einer basischen Verbindung zu dem Tallölpech;
c) Extrahieren des verseiften Tallölpechs mit einem oder mehreren unpolaren aromatischen Lösungsmitteln unter Bildung einer Lösung, die ein Gemisch von Neutralstoffen umfasst;
d) Durchführen einer Destillation des Gemischs von Neutralstoffen unter Erhalt einer Destillatfraktion und einer Sumpffraktion;
e) Lösen der Destillatfraktion in gemischten Lösungsmitteln unter Erhalt einer gelösten Destillatfraktion,

wobei das Verfahren **dadurch gekennzeichnet ist, dass** es ferner einen Kristallisationsschritt (f) umfasst, der mit der gelösten Destillatfraktion unter Kristallisierungsbedingungen durchgeführt wird, wodurch eine Mutterlauge und eine kristallisierte Fraktion gewonnen werden, wobei die kristallisierte Fraktion ein Extrakt von Phytosterolen und Stanolen bildet; und dass die in Schritt (e) verwendeten gemischten Lösungsmittel ein oder mehrere unpolare Lösungsmittel, ein oder mehrere Lösungsmittel mit einer Ketongruppierung, einen oder mehrere einwertige Alkohole und Wasser umfassen.

2. Verfahren nach Anspruch 1, **dadurch gekennzeichnet, dass** der Destillationsschritt (d) einen oder mehrere Destillationsunterschritte umfasst, so dass der Destillationsschritt (d) Folgendes umfasst:

d1) Durchführen einer ersten Destillation mit dem Gemisch von Neutralstoffen unter Erhalt einer ersten Destillatfraktion und einer ersten Sumpffraktion;
d2) Durchführen einer zweiten Destillation mit der ersten Sumpffraktion unter Erhalt einer zweiten Destillatfraktion und einer zweiten Sumpffraktion;
d3) gegebenenfalls Durchführen einer oder mehrerer weiterer Destillationen mit der zweiten Sumpffraktion und weiteren Sumpffraktionen unter Erhalt einer oder mehrerer weiterer Destillatfraktionen und einer oder mehrerer weiterer Sumpffraktionen;

wobei die Destillatfraktion, die in Schritt e) in gemischten Lösungsmitteln gelöst wird, die zweite Destillatfraktion ist oder eine Mischung der zweiten Destillatfraktion mit der ersten Destillatfraktion und/oder mindestens eine weitere Destillatfraktion der einen oder mehreren Destillatfraktionen ist.

3. Verfahren nach Anspruch 1 oder 2, **dadurch gekennzeichnet, dass** die in Schritt (e) verwendeten gemischten Lösungsmittel ein oder mehrere unpolare Lösungsmittel, die aus Pentan, Isopentan und/oder Hexane ausgewählt sind, umfassen; wobei das unpolare Lösungsmittel vorzugsweise Hexan ist oder umfasst.

4. Verfahren nach Anspruch 1 oder 3, **dadurch gekennzeichnet, dass** die in Schritt (e) verwendeten gemischten Lösungsmittel ein oder mehrere Lösungsmittel mit einer Ketongruppierung, die aus Aceton und/oder Methyletherketon ausgewählt sind, umfassen; wobei das Lösungsmittel mit einer Ketongruppierung vorzugsweise Aceton ist oder umfasst.

5. Verfahren nach einem der Ansprüche 1 bis 4, **dadurch gekennzeichnet, dass** die in Schritt (e) verwendeten gemischten Lösungsmittel einen oder mehrere einwertige Alkohole, die aus Methanol, Ethanol und/oder Propanol ausgewählt sind, umfassen; wobei der einwertige Alkohol vorzugsweise Methanol ist oder umfasst.

6. Verfahren nach einem der Ansprüche 1 bis 5, **dadurch gekennzeichnet, dass** die in Schritt (e) verwendeten gemischten Lösungsmittel Folgendes umfassen:

- 10 bis 60 Gew.-% eines oder mehrerer unpolarer Lösungsmittel, bezogen auf das Gesamtgewicht der gemischten Lösungsmittel;
- 23 bis 40 Gew.-% eines oder mehrerer Lösungsmittel mit einer Ketongruppierung, bezogen auf das Gesamtgewicht der gemischten Lösungsmittel;
- 0,5 bis 2,5 Gew.-% Wasser, bezogen auf das Gesamtgewicht der gemischten Lösungsmittel; und
- 2 bis 20 Gew.-% eines oder mehrere einwertiger Alkohole, bezogen auf das Gesamtgewicht der gemischten Lösungsmittel.

7. Verfahren nach einem der Ansprüche 1 bis 6, **dadurch gekennzeichnet, dass** in Schritt (e) das Massenverhältnis zwischen den gemischten Lösungsmitteln und der Destillatfraktion zwischen 1,0 und 2,0 liegt; wobei das Massenverhältnis vorzugsweise im Bereich von 1,1 bis 1,7 und weiter bevorzugt im Bereich von 1,2 bis 1,5 liegt.

8. Verfahren nach einem der Ansprüche 1 bis 7, **dadurch gekennzeichnet, dass** die Kristallisationsbedingungen von Schritt (f) eine Kristallisationstemperatur im Bereich zwischen 15 °C und 25 °C und/oder eine Kristallisationszeit von mindestens einer Stunde umfassen.

9. Verfahren nach einem der Ansprüche 1 bis 8, **dadurch gekennzeichnet, dass** Schritt (f) ferner einen Schritt des Durchführens einer zusätzlichen Kristallisation mit der Mutterlauge umfasst, wodurch ein zusätzlicher Extrakt von Phytosterolen und Stanolen gewonnen wird, wobei die zusätzliche Kristallisation unter den gleichen Kristallisationsbedingungen von Schritt (f) durchgeführt wird; wobei der zusätzliche Extrakt von Phytosterolen und Stanolen zu dem in Schritt (f) gewonnenen Extrakt von Phytosterolen und Stanolen gegeben wird.

10. Verfahren nach einem der Ansprüche 1 bis 9, **dadurch gekennzeichnet, dass** das Verfahren ferner Folgendes umfasst:

- einen Schritt (g) des Waschens des Extrakts von Phytosterolen und Stanolen mit den in Schritt (f) verwendeten gemischten Lösungsmitteln bei einer Temperatur zwischen 10 °C und 15 °C; und/oder
- einen zusätzlichen Schritt des Trocknens des Extrakts von Phytosterolen und Stanolen, vorzugsweise bei einer Temperatur von mindestens 100 °C über einen Zeitraum von mindestens 1 Stunde.

11. Verfahren nach einem der Ansprüche 1 bis 10, **dadurch gekennzeichnet, dass** Schritt (f) durchgeführt wird, wenn die Destillatfraktion

- einen Gehalt kleiner oder gleich 2,5 Gew.-% $\alpha$-Sitosterol, bezogen auf das Gesamtgewicht der Destillatfraktion und bestimmt durch Gaschromatographie mit Flammenionisationsdetektor-Analysen, vorzugsweise kleiner oder gleich 1,7 Gew.-% $\alpha$-Sitosterol; und/oder
- einen Gehalt von mindestens 20 Gew.-% Sterolen, bezogen auf das Gesamtgewicht der Destillatfraktion und bestimmt durch Gaschromatographie mit Flammenionisationsdetektor-Analysen, vorzugsweise mindestens 25 Gew.-%; umfasst.

**12.** Verfahren nach einem der Ansprüche 1 bis 11, **dadurch gekennzeichnet, dass** Schritt (c) des Extrahierens des verseiften Tallölpechs mit einem oder mehreren unpolaren aromatischen Lösungsmitteln bei einer Temperatur im Bereich zwischen 80 °C und einer Temperatur, die mindestens 10 °C unter der Siedetemperatur des einen oder der mehreren unpolaren aromatischen Lösungsmitteln liegt, durchgeführt wird.

**13.** Verfahren nach einem der Ansprüche 1 bis 12, **dadurch gekennzeichnet, dass** das eine oder die mehreren unpolaren Lösungsmittel, die in Schritt (c) verwendet werden, aus Toluol und/oder Xylol ausgewählt werden; wobei es sich bei dem in Schritt (c) verwendeten unpolaren aromatischen Lösungsmittel vorzugsweise um Toluol handelt.

**14.** Verfahren nach einem der Ansprüche 2 bis 13, wobei der Destillationsschritt (d) einen oder mehrere Destillationsunterschritte umfasst, wobei das Verfahren **dadurch gekennzeichnet ist, dass** es sich bei dem einen oder den mehreren Destillationsunterschritten um einen oder mehrere Rektifikationsschritte unter Verwendung einer Rektifikatioskolonne handelt; wobei der eine oder die mehreren Rektifikationschritte vorzugsweise durch die folgenden Parameter definiert sind:

- das Rücklaufverhältnis liegt zwischen 1/1 und 2/1 und/oder
- die Rektifikationstemperatur liegt zwischen 240 °C und 280 °C und/oder
- die Rektifikatioskolonne ist mit einem Rückflusskühler gekoppelt; wobei sich der Rückflusskühler vorzugsweise bei einer Temperatur zwischen 110 °C und 170 °C befindet.

**15.** Extrakt von Phytosterolen und Stanolen, erhalten aus Tallölpech, wobei der Extrakt **dadurch gekennzeichnet ist, dass** er mindestens 65 Gew.-% β-Sitosterol, bezogen auf das Gesamtgewicht des Extrakts von Phytosterolen und Stanolen, und jeweils weniger als 0,01 Gew.-%, bezogen auf das Gesamtgewicht des Extrakts von Phytosterolen und Stanolen, von Oxysterolen, α-Sitosterol und Betulin gemäß Bestimmung durch Gaschromatographie mit Flammenionisationsdetektor-Analysen umfasst.

## Revendications

**1.** Procédé d'obtention d'un extrait de phytostérols et de stanols à partir de la résine d'huile de pin, le procédé comprenant les étapes suivantes :

a) fournir une résine d'huile de pin ;
b) saponifier la résine d'huile de pin pour produire une résine d'huile de pin saponifiée par addition d'un composé basique à la résine d'huile de pin ;
c) extraire la résine d'huile de pin saponifiée avec un ou plusieurs solvants aromatiques apolaires, pour produire une solution comprenant un mélange de matières neutres ;
d) effectuer une distillation du mélange de matières neutres pour obtenir une fraction de distillat et une fraction du fond ;
e) dissoudre la fraction de distillat dans des solvants mixtes, pour obtenir une fraction de distillat dissous,

le procédé est **caractérisé en ce qu'**il comprend en outre une étape (f) de cristallisation qui est effectuée sur la fraction de distillat dissous dans des conditions de cristallisation pour récupérer une solution mère et une fraction cristallisée, la fraction cristallisée formant un extrait de phytostérols et de stanols ; et **en ce que** les solvants mixtes utilisés à l'étape (e) comprennent un ou plusieurs solvants apolaires, un ou plusieurs solvants ayant un substituant cétone, un ou plusieurs alcools monohydriques et de l'eau.

**2.** Procédé selon la revendication 1, **caractérisé en ce que** l'étape (d) de distillation comprend une ou plusieurs sousétapes de distillation de sorte que l'étape (d) de distillation comprend :

d1) effectuer une première distillation sur le mélange de matières neutres, pour obtenir une première fraction de distillat et une première fraction du fond ;
d2) effectuer une deuxième distillation sur la première fraction du fond, pour obtenir une deuxième fraction de distillat et une deuxième fraction du fond ;
d3) effectuer optionnellement une ou plusieurs autres distillations sur la deuxième fraction du fond et d'autres fractions du fond, pour obtenir une ou plusieurs autres fractions de distillat et une ou plusieurs autres fractions du fond ;

dans lequel la fraction de distillat qui est dissoute dans des solvants mixtes à l'étape e) est la deuxième fraction de distillat, ou est un mélange de la deuxième fraction de distillat avec la première fraction de distillat et/ou au moins une autre fraction de distillat à partir d'une ou plusieurs fractions de distillat.

3. Procédé selon l'une quelconque des revendications 1 ou 2, **caractérisé en ce que** les solvants mixtes utilisés à l'étape (e) comprennent un ou plusieurs solvants apolaires choisis parmi le pentane, l'isopentane et/ou l'hexane ; de préférence, le solvant apolaire est ou comprend de l'hexane.

4. Procédé selon les revendications 1 ou 3, **caractérisé en ce que** les solvants mixtes utilisés à l'étape (e) comprennent un ou plusieurs solvants ayant un substituant cétone choisi parmi l'acétone et/ou la méthyl éther cétone ; de préférence, le solvant ayant un substituant cétone est ou comprend de l'acétone.

5. Procédé selon l'une quelconque des revendications 1 à 4, **caractérisé en ce que** les solvants mixtes utilisés à l'étape (e) comprennent un ou plusieurs alcools monohydriques choisis parmi le méthanol, l'éthanol et/ou le propanol ; de préférence, l'alcool monohydrique est ou comprend du méthanol.

6. Procédé selon l'une quelconque des revendications 1 à 5, **caractérisé en ce que** les solvants mixtes utilisés à l'étape (e) comprennent :

   - de 10 à 60 % en poids d'un ou plusieurs solvants apolaires sur la base du poids total des solvants mixtes ;
   - de 23 à 40 % en poids d'un ou plusieurs solvants ayant un substituant cétone sur la base du poids total des solvants mixtes ;
   - de 0,5 à 2,5 % en poids d'eau sur la base du poids total des solvants mixtes, et
   - de 2 à 20 % en poids d'un ou plusieurs alcools monohydriques sur la base du poids total des solvants mixtes.

7. Procédé selon l'une quelconque des revendications 1 à 6, **caractérisé en ce qu'**à l'étape (e) le rapport de masse entre les solvants mixtes et la fraction de distillat est compris entre 1,0 et 2,0 ; de préférence, le rapport de masse est compris entre 1,1 et 1,7 ; plus préférablement est compris entre 1,2 à 1,5.

8. Procédé selon l'une quelconque des revendications 1 à 7, **caractérisé en ce que** les conditions de cristallisation de l'étape (f) comprennent une température de cristallisation comprise entre 15°C et 25°C et/ou une durée de cristallisation d'au moins une heure.

9. Procédé selon l'une quelconque des revendications 1 à 8, **caractérisé en ce que** l'étape (f) comprend en outre une étape consistant à effectuer une cristallisation supplémentaire sur la solution mère, pour récupérer un extrait supplémentaire de phytostérols et de stanols, la cristallisation supplémentaire étant réalisée sous les mêmes conditions de cristallisation de l'étape (f) ; de préférence, l'extrait supplémentaire de phytostérols et de stanols est ajouté à l'extrait de phytostérols et de stanols récupéré à l'étape (f).

10. Procédé selon l'une quelconque des revendications 1 à 9, **caractérisé en ce que** le procédé comprend en outre

    - une étape (g) de lavage de l'extrait de phytostérols et de stanols avec les solvants mixtes utilisés à l'étape (f) à une température comprise entre 10°C et 15°C ; et/ou
    - une étape supplémentaire de séchage de l'extrait de phytostérols et de stanols, préférentiellement à une température d'au moins 100°C pendant au moins 1 heure.

11. Procédé selon l'une quelconque des revendications 1 à 10, **caractérisé en ce que** l'étape (f) est réalisée lorsque la fraction de distillat comprend

    - une teneur inférieure ou égale à 2,5 % en poids d'$\alpha$-sitostérol sur la base du poids total de la fraction de distillat et déterminée par chromatographie en phase gazeuse avec analyses au détecteur à ionisation de flamme, de préférence inférieure ou égale à 1,7 % en poids d'$\alpha$- sitostérol ; et/ou
    - une teneur d'au moins 20 % en poids de stérols sur la base du poids total de la fraction de distillat et déterminée par chromatographie en phase gazeuse avec analyses au détecteur à ionisation de flamme, de préférence d'au moins 25 % en poids.

**12.** Procédé selon l'une quelconque des revendications 1 à 11, **caractérisé en ce que** l'étape (c) d'extraction de la résine d'huile de pin saponifiée par un ou plusieurs solvants aromatiques apolaires est réalisée à une température comprise entre 80°C et une température au moins 10°C inférieure à la température d'ébullition du ou des solvants aromatiques apolaires.

**13.** Procédé selon l'une quelconque des revendications 1 à 12, **caractérisé en ce que** le ou les solvants aromatiques apolaires utilisés à l'étape (c) sont choisis parmi le toluène et/ou le xylène ; de préférence, le solvant aromatique apolaire utilisé à l'étape (c) est le toluène.

**14.** Procédé selon l'une quelconque des revendications 2 à 13, dans lequel l'étape (d) de distillation comprend une ou plusieurs sous-étapes de distillation, le procédé est **caractérisé en ce que** la ou les sous-étapes de distillation sont une ou plusieurs étapes de rectification, utilisant une colonne de rectification ; de préférence, les paramètres suivants définissant le ou les étapes de rectification

    - le taux de reflux est compris entre 1/1 et 2/1 ; et/ou
    - la température de rectification est comprise entre 240°C et 280°C, et/ou
    - la colonne de rectification est couplée à un condenseur à reflux ; de préférence, le condenseur à reflux est à une température comprise entre 110°C et 170°C.

**15.** Extrait de phytostérols et de stanols obtenu à partir de la résine d'huile de pin, l'extrait étant **caractérisé en ce qu'**il comprend au moins 65 % en poids de $\beta$-sitostérol sur la base du poids total de l'extrait de phytostérols et de stanols, et moins de 0,01 % en poids sur la base du poids total de l'extrait de phytostérols et de stanols de chacun des oxystérols, $\alpha$-sitostérol et la bétuline comme déterminé par chromatographie en phase gazeuse avec analyses au détecteur à ionisation de flamme.

**Fig. 1**

**Fig. 2**

23

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6465665 B
- US 3965085 A
- WO 2019050430 A
- US 20080161586 A
- US 2011034725 A
- US 2004267032 A
- US 2004024175 A
- US 4279827 A
- US 2013261284 A